# EUROPÄISCHE PATENTSCHRIFT

(11) **EP 2 013 144 B1**
(45) Veröffentlichungstag und Bekanntmachung des Hinweises auf die Patenterteilung: **03.10.2012**
(21) Anmeldenummer: 07728038.6
(22) Anmeldetag: 12.04.2007
(51) Int. Cl.: C01G 1/02, C04B 35/486, C01G 25/02, C01G 23/053, C01F 7/02, C01F 5/02, C01F 7/30, C04B 35/111, C04B 35/053, C04B 38/00, C04B 35/10, C04B 35/04, C04B 35/48

(54) **METALLOXIDE AUS METALLORGANISCHEN GERÜSTMATERIALIEN**
METAL OXIDES PRODUCED FROM METAL-ORGANIC FRAMEWORK MATERIALS
OXYDES MÉTALLIQUES OBTENUS À PARTIR DE MATÉRIAUX STRUCTURANTS ORGANO-MÉTALLIQUES

(30) Priorität: 18.04.2006 EP 06112713
(43) Veröffentlichungstag der Anmeldung: 14.01.2009
(73) Patentinhaber: BASF SE, 67056 Ludwigshafen (DE)
(72) Erfinder: SCHUBERT, Markus, 67063 Ludwigshafen (DE); MUELLER, Ulrich, 67435 Neustadt (DE); TRUKHAN, Natalia, 67063 Ludwigshafen (DE)
(86) Internationale Anmeldenummer: PCT/EP2007/053571
(87) Internationale Veröffentlichungsnummer: WO 2007/118843

(56) Entgegenhaltungen:
- EP-A- 1 595 847
- EP-A1- 0 370 728
- EP-A1- 0 449 672
- WO-A-99/50203
- DE-A1- 10 226 131
- US-A- 4 656 156
- US-A- 5 922 294
- US-A- 6 139 814
- US-A1- 2004 065 619

## Beschreibung

Die vorliegende Erfindung betrifft Verfahren zur Herstellung von Metalloxiden aus porösen metallorganischen Gerüstmaterialien, Metalloxiden, die aus diesen Verfahren erhältlich sind sowie deren Verwendung.

Metalloxide stellen eine interessante Stoffgruppe dar und werden für zahlreiche Anwendungen eingesetzt.

Nicht zuletzt aus diesem Grund existieren im Stand der Technik zahlreiche Methoden, um solche Metalloxide zu erhalten. Neben der Gewinnung beispielsweise aus Erzen stellt die großtechnische Darstellung insbesondere hoch-reiner Metalloxide eine Möglichkeit dar.

Ausgangsstoffe solcher Darstellungsmethoden können unterschiedlicher Natur sein. So wird beispielsweise α-Aluminiumoxid aus Bauxit hergestellt. γ-Al₂O₃, das für seine Porosität bekannt ist; kann beispielsweise aus Hydrargillit oder Böhmit dargestellt werden, wobei bei einer Temperatur oberhalb von 1000 °C das γ-Al₂O₃ in α-Al₂O₃ übergeht. γ-Al₂O₃ dient beispielsweise als Adsorbens, Katalysator oder Katalysatorträger.

Zur Herstellung von Metalloxiden werden auch metallorganische Verbindungen eingesetzt. Die Darstellung der Metalloxide kann hierbei beispielsweise mit Hilfe einer chemischen Gasphasenabscheidung erzeugt werden. Hierdurch können insbesondere Schichten auf entsprechenden Substraten gebildet werden. Darüber hinaus sind Sol-Gel-Verfahren, Gefriertrocknung oder die Herstellung durch Flammenpyrolyse bekannt.

Ein interessanter Ansatz zur Darstellung von Zinkoxid ist von C.-Y. Su et al., J. Am. Chem. Soc. 126 (2004), 3576-3586 beschrieben. Hierbei werden zunächst metallorganische Gerüstmaterialien hergestellt, die zum einen aus einem Metallion (Zink) und einem organischen Liganden (3-Amino-1,2,4-triazol oder 3-Amino-1,2,4-triazol-5-carbonsäure) ein poröses dreidimensionales metallorganisches Gerüst bilden. Das Erhitzen des Gerüstmaterials in Sauerstoffatmosphäre führt bei Temperaturen oberhalb 600°C zur Bildung von Zinkoxid.

Poröse metallorganische Gerüstmaterialien, welche auf Zink basieren, sind in der Literatur bekannt und zeichnen sich durch eine vergleichsweise hohe spezifische Oberfläche aus, so dass diese Materialien häufig vorgeschlagen werden, um beispielsweise Adsorptionen von Gasen durchzuführen. Hierbei ist Zinkterephthalat (MOF-5) am bekanntesten.

Es ist im Stand der Technik jedoch nicht bekannt, ob das aus einem metallorganischen Gerüstmaterial gebildete Zinkoxid Eigenschaften aufweist, die die Verwendung eines solchen Oxids als vielversprechend darstellen lassen.

Eine Aufgabe der vorliegenden Erfindung liegt somit darin, Verfahren zur Herstellung von Metalloxiden sowie solche Metalloxide bereitzustellen, um diese einer geeigneten Anwendung zuzuführen.

Die Aufgabe wird gelöst durch ein Verfahren zur Herstellung eines Metalloxids den Schritt enthaltend
- Erhitzen eines porösen metallorganischen Gerüstmaterials, wobei das Gerüstmaterial mindestens eine an mindestens ein Metallion koordinativ gebundene, mindestens zweizähnige organische Verbindung enthält und das Metallion ausgewählt ist aus den Metallen bestehend aus den Gruppen 2. bis 4. und 13. des Periodensystems der Elemente, über die vollständige Zersetzungstemperatur des Gerüstmaterials.

Es wurde nämlich gefunden, dass die Oxide der oben genannten Metalle der 2. bis 4. und 13. Gruppe, welche nach dem erfindungsgemäßen Verfahren hergestellt werden, eine vergleichsweise hohe spezifische Oberfläche aufweisen und so insbesondere in Verwendungsgebieten der Adsorption von Stoffen einsetzbar sind. Diese gegenüber dem Stand der Technik vergleichsweise hohen spezifischen Oberflächen könnten dadurch erklärt werden, dass die Gerüststruktur des metallorganischen Gerüstmaterials zumindest teilweise in der Struktur des entsprechenden Metalloxids beibehalten wird.

Überraschenderweise hat sich jedoch herausgestellt, dass das im Stand der Technik bekannte Zinkoxid aus einem metallorganischen Gerüstmaterial sehr niedrige spezifische Oberflächen aufweist und somit nicht in besonderem Maße geeignet ist, entsprechenden Verwendungen zugeführt zu werden. Es ist daher umso überraschender, dass dies mit den erfindungsgemäßen Metallen möglich ist.

Das erfindungsgemäße Verfahren zur Herstellung eines Metalloxids geht somit von einem Schritt aus, bei dem das poröse metallorganische Gerüstmaterial erhitzt wird. Hierbei kann das Material in einer Dispersion oder als trockener Feststoff vorliegen. Weiterhin kann das metallorganische Gerüstmaterial als Pulver oder als Formkörper oder beides vorliegen. Vorzugsweise erfolgt das Erhitzen mit einem porösen metallorganischen Gerüstmaterial in Form eines Pulvers.

Das Erhitzen kann durch für den Fachmann bekannte Methoden erfolgen. Typischerweise erfolgt das Erhitzen in einem dazu geeigneten Ofen, wie beispielsweise einem Muffelofen oder Drehrohrofen. Bei Verwendung eines Ofens ist es weiterhin zweckdienlich, dass Möglichkeiten vorhanden sind, das Erhitzen in Gegenwart einer geeigneten Atmosphäre durchführen zu können. Hierzu kann entsprechend eine Zuführung für ein entsprechendes Gas oder Gasgemisch derart im oder am Ofen angebracht sein, damit der das poröse metallorganische Gerüstmaterial enthaltende Ofenraum mit dem entsprechenden Gas oder Gasgemisch geflutet werden kann.

Das poröse metallorganische Gerüstmaterial wird soweit erhitzt, wie es erforderlich ist, um das metallorganische Gerüstmaterial in das entsprechende Metalloxid umzuwandeln. Hierbei wird daher über die vollständige Zersetzungstemperatur des metallorganischen Gerüstmaterials erhitzt.

Im Rahmen der vorliegenden Erfindung ist unter "vollständige Zersetzungstemperatur" diejenige Temperatur zu verstehen, bei der das poröse metallorganische Gerüstmaterial beginnt, sich in das entsprechende Metalloxid umzuwandeln. Es ist jedoch ebenfalls möglich, dass das metallorganische Gerüstmaterial über Zwischenstufen zum Metalloxid umgewandelt wird. Beispielsweise könnte vor Bildung des Metalloxids ein Carbonat gebildet worden sein. In einem solchen Fall ist unter der "vollständigen Zersetzungstemperatur" diejenige Temperatur zu verstehen, die erforderlich ist, die jeweils letzte Zwischenstufe zum Metalloxid zu überführen.

Die Bestimmung der vollständigen Zersetzungstemperatur kann anhand der vom Fachmann bekannten Methoden durchgeführt werden. Beispielsweise kann durch Thermogravimetrie diese Temperatur bestimmt werden, wobei durch begleitende Analytik ebenfalls der Nachweis der Bildung des entsprechenden Metalloxids geführt werden kann.

Die vollständige Zersetzungstemperatur, die erforderlich ist, aus einem porösen metallorganischen Gerüstmaterial das entsprechende Metalloxid herzustellen, liegt typischerweise im Bereich von 250°C bis 1000°C. Weiterhin bevorzugt liegt die vollständige Zersetzungstemperatur in einem Bereich von 350°C bis 800°C. Insbesondere bevorzugt liegt die vollständige Zersetzungstemperatur im Bereich von 450°C bis 650°C. Liegt das Metalloxid in verschiedenen Modifikationen vor, die durch Temperaturbehandlung erhalten werden können, kann die thermisch höherstufige(n) Modifikation(en) aus dem metallorganischen Gerüstmaterial durch Anlegen der entsprechenden Temperaturstufe erhalten werden oder es wird zunächst das/die tieferstufige(n) Modifikation(en) erhalten und in einem weiteren Schritt kann dann die Umwandlung in die gewünschte Modifikation erfolgen. '

Wie bereits oben erwähnt wurde, kann das Erhitzen des porösen metallorganischen Gerüstmaterials in einer geeigneten Atmosphäre stattfinden. Sofern das poröse metallorganische Gerüstmaterial mindestens eine mindestens zweizähnige organische Verbindung enthält, die selbst genügend Sauerstoff aufweist, ist es nicht zwingend erforderlich, dass von außen eine Sauerstoff liefernde Substanz zur Verfügung gestellt wird, um das poröse metallorganische Gerüstmaterial in ein Metalloxid umzuwandeln. Beispiele solcher mindestens zweizähniger organischer Verbindungen, die Sauerstoff enthalten, sind Carbonsäuren, Alkohole, Ketone, Aldehyde, Ether, Ester und Phenole. Insofern könnte das Erhitzen des porösen metallorganischen Gerüstmaterials im Vakuum erfolgen. Zweckdienlicherweise wird jedoch das Erhitzen unter Atmosphärenbedingungen durchgeführt. In einem solchen Falle könnte also auch das Erhitzen des porösen metallorganischen Gerüstmaterials in Gegenwart einer inerten Atmosphäre stattfinden. Solche Atmosphären könnten gebildet sein durch Gase wie Stickstoff, E-delgase wie Helium oder Argon und Gemische hiervon. Dies stellt jedoch eine Ausnahme dar.

Vorzugsweise findet daher jedoch das Erhitzen des porösen metallorganischen Gerüstmaterials in Gegenwart einer oxidierenden Atmosphäre mit einem Sauerstoff liefernden Bestandteil statt. Hierdurch kann gewährleistet werden, dass für die Umwandlung des porösen metallorganischen Gerüstmaterials in das entsprechende Metalloxid ausreichend Sauerstoff zur Verfügung steht. Dies kann insbesondere auch dazu beitragen, dass die oben erwähnten Zwischenstufen "übersprungen" werden. Solche oxidierenden Atmosphären können durch entsprechend Sauerstoff liefernde Gase oder Gasgemische erhalten werden. Als einfachstes und bevorzugtes Gasgemisch ist hierbei Luft zu nennen, das normalerweise einen ausreichend hohen Anteil an molekularem Sauerstoff enthält. Gegebenenfalls kann die Luft mit weiterem Sauerstoff angereichert eingesetzt werden. Schließlich ist es selbstverständlich ebenso möglich, dass reiner Sauerstoff als oxidierende Atmosphäre verwendet wird. Darüber hinaus können auch andere Gase oder Gasgemische verwendet werden, die beispielsweise mit molekularem Sauerstoff angereichert sind. Hierbei wären insbesondere Inertgase bevorzugt. So können geeignete Gasgemische zur Erzeugung einer oxidierenden Atmosphäre bei Erhitzen des porösen metallorganischen Gerüstmaterials Helium, Argon, Stickstoff oder Gemische hiervon jeweils mit Sauerstoff angereichert eingesetzt werden.

Das poröse metallorganische Gerüstmaterial kann einer oxidierenden Atmosphäre derart ausgesetzt sein, dass während des Erhitzens die Atmosphäre nicht verändert wird. Das das poröse metallorganische Gerüstmaterial umgebende Gas oder Gasgemisch wird somit nicht ausgetauscht, so dass der Sauerstoff liefernde Bestandteil der Atmosphäre während des Erhitzens abnimmt.

Darüber hinaus ist es möglich, die Atmosphäre während des Erhitzens in Bezug auf deren Sauerstoff liefernden Bestandteil durch Nachführen zumindest dieses Bestandteils in etwa konstant zu halten.

Bevorzugt ist jedoch, dass der Sauerstoff liefernde Bestandteil während des Erhitzens erhöht wird. Dies kann zur Temperatursteuerung der exothermen Reaktion dienen. Eine mögliche Ausführungsform ist, dass die Atmosphäre durch ein Gas oder Gasgemisch mit höherem Anteil an Sauerstoff lieferndem Bestandteil ausgetauscht wird. Dies kann insbesondere derart erfolgen, dass der Atmosphäre nach dem Beginn des Erhitzens Sauerstoff zugeführt wird, bis schließlich eine bestimmte Sauerstoffatmosphäre vorliegt. Die Erhöhung kann schrittweise oder kontinuierlich erfolgen.

Das poröse metallorganische Gerüstmaterial für das erfindungsgemäße Verfahren zur Herstellung eines Metalloxids muss das dem Metall des Metalloxids entsprechende Metallion enthalten. Das poröse metallorganische Gerüstmaterial kann jedoch auch mehrere Metallionen enthalten. Hierbei entsteht dann entsprechend ein Metalloxid, das ebenfalls aus mehreren Metallen aufgebaut ist.

Für den Fall, dass mehrere Metallionen im metallorganischen Gerüstmaterial vorhanden sind, muss mindestens eines dieser Metallionen fähig sein, die mindestens eine mindestens zweizähnige organische Verbindung koordinativ zu binden, um das entsprechende poröse metallorganische Gerüstmaterial zu erhalten. Sind darüber hinaus ein oder mehrere Metalle in ionischer Form vorhanden, kann dieses oder können diese ebenfalls durch Koordination der mindestens einen mindestens zweizähnigen organischen Verbindungen oder weiterer mindestens zweizähniger organischer Verbindungen am Aufbau des metallorganischen Gerüstmaterials vorhanden sein. Darüber hinaus ist es jedoch ebenso möglich, dass dies nicht der Fall ist. Schließlich kann bei Vorhandensein mehrerer Metallionen das Verhältnis der Ionen in einem stöchiometrischen Verhältnis gegeben sein. Darüber hinaus kann auch ein nicht stöchiometrisches Verhältnis vorliegen. In diesem Zusammenhang kann dann auch von einem sogenannten dotieren porösen metallorganischen Gerüstmaterial ausgegangen werden. Solche dotierten Gerüstmaterialien sind beispielsweise in der deutschen Patentanmeldung mit der Anmeldenummer 10 2005 053 430.0 der Anmelderin beschrieben. Solche dotierten porösen metallorganischen Gerüstmaterialien zeichnen sich dadurch aus, dass die Verteilung des Dotierungsmetalls zufällig erfolgt.

Darüber hinaus kann das poröse metallorganische Gerüstmaterial ebenso durch ein weiteres Metall beispielsweise in Form eines Salzes imprägniert sein. Ein Verfahren zum Imprägnieren ist zum Beispiel in EP-A 1 070 538 beschrieben.

Im Rahmen der vorliegenden Erfindung gelten zwei Metallionen ein und desselben Metalls unterschiedlicher Oxidationsstufe als zwei unterschiedliche Metallionen. Hierbei kann also ein entsprechendes Metalloxid erhalten werden, bei dem das Metall in unterschiedlichen Oxidationsstufen vorliegt. Vorzugsweise wird jedoch insbesondere in Gegenwart einer oxidierenden Atmosphäre ein solches Metall ausschließlich in der höchsten stabilen Oxidationsstufe als Metalloxid vorliegen.

Im Rahmen der vorliegenden Erfindung ist es bevorzugt, wenn das poröse metallorganische Gerüstmaterial ausschließlich ein Metallion eines Metalls, insbesondere einer Oxidationsstufe, aufweist.

Das oder die Metallionen sind ausgewählt aus den Metallen bestehend aus den Gruppen 2. bis 4. und 13. des Periodensystems der Elemente.

Insbesondere geeignete Metalle der Gruppe 2. des Periodensystems der Elemente sind Beryllium, Magnesium, Calcium, Strontium sowie Barium.

Insbesondere geeignete Metalle der 3. Gruppe des Periodensystems der Elemente sind Scandium, Ytrium, Lanthan sowie die Lanthanide.

Insbesonders geeignete Metalle der 4. Gruppe des Periodensystems der Elemente sind Titan, Zirkonium sowie Hafnium.

Insbesondere geeignete Metalle der 13. Gruppe des Periodensystems der Elemente sind Aluminium, Bor, Gallium sowie Indium.

Weiterhin bevorzugt sind die Metalle Magnesium, Calcium, Strontium, Barium, Zirkonium sowie Aluminium.

ganz besonders bevorzugt ist das Metallion beziehungsweise die Metallionen aus der Gruppe der Metalle bestehend aus Aluminium, Magnesium und Zirkonium.

Für den Fall, dass mehr als ein Metallion in porösen metallorganischen Gerüstmaterialien vorhanden ist, können insbesondere Aluminate der Formel M^{I}AlO₂ oder M^{II}Al₂O₄ erhalten werden, wobei M^{I} ein einwertiges Metallion darstellt und M" ein zweiwertiges Metallion ist. Insbesondere können Spinelle erhalten werden.

Für den Fall, dass das metallorganische Gerüstmaterial neben Titan weitere Metalle enthält, ist es möglich, Titanate, insbesondere Ilmenit (FeTiO₃) aber auch MgTiO₃, MnTiO₃ FeTiO₃, CoTiO₃, NiTiO₃, CaTiO₃, SrTiO₃, BaTiO₃, Mg₂TiO₄, Zn₂TiO₄ sowie Mn₂TiO₄ zu erhalten.

Bei Verwendung von Zirkon im metallorganischen Gerüstmaterial und darüber hinaus wenigstens einem weiteren Metallion können entsprechende Zirkonate erhalten werden.

Geeignete poröse metallorganische Gerüstmaterialien sind aus dem Stand der Technik bekannt oder können anhand der im Stand der Technik beschriebenen Verfahren analog hergestellt werden. Metallorganische Gerüstmaterialien sind beispielsweise beschrieben in US 5,648,508, EP-A-0 790 253, M.O. Keeffe et al., J. Sol. State Chem., 152 (2000), Seite 3 bis 20, H. Li et al., Nature 402, (1999), Seite 276, M. Eddaoudi et al., Topics in Catalysis 9, (1999), Seite 105 bis 111, B. Chen et al., Science 291, (2001), Seite 1021 bis 1023, DE-A-101 11 230, WO-A 2005/049892 und A.C. Sudik et al., J. Am. Chem. Soc. 127 (2005), 7110 bis 7118.

Poröse metallorganische Gerüstmaterialien basierend auf Aluminium und Magnesium sind darüber hinaus insbesondere in DE A 10 2005 039 623 sowie der darin zitierten Literatur beschrieben.

Poröse metallorganische Gerüstmaterialien basierend auf Zirkonium und Titan sind insbesondere in der parallel eingereichten europäischen Patentanmeldung der Anmelderin mit dem Anwaltsaktenzeichen B06/0128EP beschrieben.

Der Begriff "mindestens zweizähnige organische Verbindung" bezeichnet eine organische Verbindung, die mindestens eine funktionelle Gruppe enthält, die in der Lage ist, zu einem gegebenen Metallion mindestens zwei, bevorzugt zwei koordinative Bindungen, und/oder zu zwei oder mehr, bevorzugt zwei Metallatomen jeweils eine koordinative Bindung auszubilden.

Als funktionelle Gruppen, über die die genannten koordinativen Bindungen ausgebildet werden kann, sind insbesondere beispielsweise folgende funktionellen Gruppen zu nennen: -CO₂H, -CS₂H, -NO₂, -B(OH)₂, -SO₃H, -Si(OH)₃, -Ge(OH)₃, -Sn(OH)₃, -Si(SH)₄, -Ge(SH)₄, -Sn(SH)₃, -PO₃H, -AsO₃H, -AsO₄H, -P(SH)₃, -As(SH)₃, -CH(RSH)₂, -C(RSH)₃ -CH(RNH₂)₂ -C(RNH₂)₃, -CH(ROH)₂, -C(ROH)₃, -CH(RCN)₂, -C(RCN)₃ wobei R beispielsweise bevorzugt eine Alkylengruppe mit 1, 2, 3, 4 oder 5 Kohlenstoffatomen wie beispielsweise eine Methylen-, Ethylen-, n-Propylen-, i-Propylen, n-Butylen-, i-Butylen-, tert-Butylen- oder n-Pentylengruppe, oder eine Arylgruppe, enthaltend 1 oder 2 aromatische Kerne wie beispielsweise 2 C₆-Ringe, die gegebenenfalls kondensiert sein können und unabhängig voneinander mit mindestes jeweils einem Substituenten geeignet substituiert sein können, und/oder die unabhängig voneinander jeweils mindestens ein Heteroatom wie beispielsweise N, O und/oder S enthalten können. Gemäß ebenfalls bevorzugter Ausführungsformen sind funktionelle Gruppen zu nennen, bei denen der oben genannte Rest R nicht vorhanden ist. Diesbezüglich sind unter anderem -CH(SH)₂, -C(SH)₃, -CH(NH₂)₂, -C(NH₂)₃, -CH(OH)₂, -C(OH)₃, -CH(CN)₂ oder -C(CN)₃ zu nennen.

Die mindestens zwei funktionellen Gruppen können grundsätzlich an jede geeignete organische Verbindung gebunden sein, solange gewährleistet ist, dass die diese funktionellen Gruppen aufweisende organische Verbindung zur Ausbildung der koordinativen Bindung und zur Herstellung des Gerüstmaterials befähigt ist.

Bevorzugt leiten sich die organischen Verbindungen, die die mindestens zwei funktionellen Gruppen enthalten, von einer gesättigten oder ungesättigten aliphatischen Verbindung oder einer aromatischen Verbindung oder einer sowohl aliphatischen als auch aromatischen Verbindung ab.

Die aliphatische Verbindung oder der aliphatische Teil der sowohl aliphatischen als auch aromatischen Verbindung kann linear und/oder verzweigt und/oder cyclisch sein, wobei auch mehrere Cyclen pro Verbindung möglich sind. Weiter bevorzugt enthält die aliphatische Verbindung oder der aliphatische Teil der sowohl aliphatischen als auch aromatischen Verbindung 1 bis 15, weiter bevorzugt 1 bis 14, weiter bevorzugt 1 bis 13, weiter bevorzugt 1 bis 12, weiter bevorzugt 1 bis 11 und insbesondere bevorzugt 1 bis 10 C-Atome wie beispielsweise 1, 2, 3, 4, 5, 6, 7, 8, 9 oder 10 C-Atome. Insbesondere bevorzugt sind hierbei unter anderem Methan, Adamantan, Acetylen, Ethylen oder Butadien.

Die aromatische Verbindung oder der aromatische Teil der sowohl aromatischen als auch aliphatischen Verbindung kann einen oder auch mehrere Kerne wie beispielsweise zwei, drei, vier oder fünf Kerne aufweisen, wobei die Kerne getrennt voneinander und/oder mindestens zwei Kerne in kondensierter Form vorliegen können. Besonders bevorzugt weist die aromatische Verbindung oder der aromatische Teil der sowohl aliphatischen als auch aromatischen Verbindung einen, zwei oder drei Kerne auf, wobei einer oder zwei Kerne besonders bevorzugt sind. Unabhängig voneinander kann weiter jeder Kern der genannten Verbindung mindestens ein Heteroatom wie beispielsweise N, O, S, B, P, Si, Al, bevorzugt N, O und/oder S enthalten. Weiter bevorzugt enthält die aromatische Verbindung oder der aromatische Teil der sowohl aromatischen als auch aliphatischen Verbindung einen oder zwei C₆-Kerne, wobei die zwei entweder getrennt voneinander oder in kondensierter Form vorliegen. Insbesondere sind als aromatische Verbindungen Benzol, Naphthalin und/oder Biphenyl und/oder Bipyridyl und/oder Pyridyl zu nennen.

Besonders bevorzugt leitet sich die mindestens zweizähnige organische Verbindung von einer Di-, Tri-, oder Tetracarbonsäure oder deren Schwefelanaloga ab. Schwefelanaloga sind die funktionellen Gruppen -C(=O)SH sowie dessen Tautomer und C(=S)SH, die anstelle einer oder mehrerer Carbonsäuregruppen eingesetzt werden können.

Der Begriff "ableiten" bedeutet im Rahmen der vorliegenden Erfindung, dass die mindestens zweizähnige organische Verbindung im Gerüstmaterial in teilweise deprotonierter oder vollständig deprotonierter Form vorliegen kann. Weiterhin kann die mindestens zweizähnige organische Verbindung weitere Substituenten enthalten, wie beispielsweise -OH, -NH₂, -OCH₃, -CH₃, -NH(CH₃), -N(CH₃)₂, -CN sowie Halogenide.

Beispielsweise sind im Rahmen der vorliegenden Erfindung Dicarbonsäuren wie etwa Oxalsäure, Bernsteinsäure, Weinsäure, 1,4-Butandicarbonsäure, 4-Oxo-Pyran-2,6-dicarbonsäure, 1,6-Hexandicarbonsäure, Decandicarbonsäure, 1,8-Heptadecandicarbonsäure, 1,9-Heptadecandicarbonsäure, Heptadecandicarbonsäure, Acetylendicarbonsäure, 1,2-Benzoldicarbonsäure, 2,3-Pyridindicarbonsäure, Pyridin-2,3-dicarbonsäure, 1,3-Butadien-1,4-dicarbonsäure, 1,4-Benzoldicarbonsäure, p-Benzoldicarbonsäure, Imidazol-2,4-dicarbonsäure, 2-Methylchinolin-3,4-dicarbonsäure, Chinolin-2,4-dicarbonsäure, Chinoxalin-2,3-dicarbonsäure, 6-Chlorchinoxalin-2,3-dicarbonsäure, 4,4'-Diaminphenylmethan-3,3'-dicarbonsäure, Chinolin-3,4-dicarbonsäure, 7-Chlor-4-hydroxychinolin-2,8-dicarbonsäure, Diimiddicarbonsäure, Pyridin-2,6-dicarbonsäure, 2-Methylimidazol-4,5-dicarbonsäure, Thiophen-3,4-dicarbonsäure, 2-Isopropylimidazol-4,5-dicarbonsäure, Tetrahydropyran-4,4-dicarbonsäure, Perylen-3,9-dicarbonsäure, Perylendicarbonsäure, Pluriol E 200-dicarbonsäure, 3,6-Dioxaoctandicarbonsäure, 3,5-Cyclohexadien-1,2-dicarbonsäure, Octadicarbonsäure, Pentan-3,3-carbonsäure, 4,4'-Diamino-1,1'-diphenyl-3,3'-dicarbon-säure, 4,4'-Diaminodiphenyl-3,3'-dicarbonsäure, Benzidin-3,3'-dicarbonsäure, 1,4-bis-(Phenylaminofbenzol-2,5-dicarbonsäure, 1,1'-Dinaphthyl-5,5'-dicarbonsäure, 7-Chlor-8-methylchinolin-2,3-dicarbonsäure, 1-Anilinoanthrachinon-2,4'-dicarbonsäure, Polytetrahydrofuran-250-dicarbonsäure, 1,4-bis-(Carboxymethyl)-piperazin-2,3-dicarbonsäure, 7-Chlorchinolin-3,8-dicarbonsäure, 1-(4-Carboxy)-phenyl-3-(4-chlor)-phenylpyrazolin-4,5-dicarbonsäure, 1,4,5,6,7,7,-Hexachlor-5-norbornen-2,3-dicarbonsäure, Phenylindandicarbonsäure, 1,3-Dibenzyl-2-oxo-imidazolidin-4,5-dicarbonsäure, 1,4-Cyclohexandicarbonsäure, Naphthalin-1,8-dicarbonsäure, 2-Benzoylbenzol-1,3-dicarbonsäure, 1,3-Dibenzyl-2-oxoimidazolidin-4,5-cis-dicarbonsäure, 2,2'-Bichinolin-4,4'-dicarbonsäure, Pyridin-3,4-dicarbonsäure, 3,6,9-Trioxaundecandicarbonsäure, O-Hydroxybenzophenondicarbonsäure, Pluriol E 300-dicarbonsäure, Pluriol E 400-dicarbonsäure, Pluriol E 600-dicarbonsäure, Pyrazol-3,4-dicarbonsäure, 2,3-Pyrazindicarbonsäure, 5,6-Dimethyl-2,3-pyrazindicarbonsäure, 4,4'-Diaminodiphenyletherdiimiddicarbonsäure, 4,4'-Diaminodiphenylmethandiimiddicarbonsäure, 4,4'-Diaminodiphenylsulfondümiddicarbonsäure, 2,6-Naphthalindicarbonsäure, 1,3-Adamantandicarbonsäure, 1,8-Naphthalindicarbonsäure, 2,3-Naphthalindicarbonsäure, 8-Methoxy-2,3-naphthalindicarbonsäure, 8-Nitro-2,3-naphthalincarbonsäure, 8-Sulfo-2,3-naphthalindicarbonsäure, Anthracen-2,3-dicarbonsäure, 2',3'-Diphenyl-p-terphenyl-4,4"-dicarbonsäure, Diphenylether-4,4'-dicarbonsäure, Imidazol-4,5-dicarbonsäure, 4(1H)-Oxothiochromen-2,8-dicarbonsäure, 5-tert-Butyl-1,3-benzöldicarbonsäure, 7,8-Chinolindicarbonsäure, 4,5-Imidazoldicarbonsäure, 4-Cyclohexen-1,2-dicarbonsäure, Hexatriacontandicarbonsäure, Tetradecandicarbonsäure, 1,7-Heptadicarbonsäure, 5-Hydroxy-1,3-Benzoldicarbonsäure, Pyrazin-2,3-dicarbonsäure, Furan-2,5-dicarbonsäure, 1-Nonen-6,9-dicarbonsäure, Eicosendicarbonsäure, 4,4'-Dihydroxydiphenylmethan-3,3'-dicarbonsäure, 1-Amino-4-methyl-9,10-dioxo-9,10-dihydroanthracen-2,3-dicarbonsäure, 2,5-Pyridindicarbonsäure, Cyclohexen-2,3-dicarbonsäure,2,9-Dichlorfluorubin-4,11-dicarbonsäure, 7-Chlor-3-mtehylchinolin-6,8-dicarbonsäure, 2,4-Dichlorbenzophenon-2',5'-dicarbonsäure, 1,3-benzoldicarbonsäure, 2,6-Pyridindicarbonsäure, 1-Methylpyrrol-3,4-dicarbonsäure, 1-Benzyl-1H-pyrrol-3,4-dicarbonsäure, Anthrachinon-1,5-dicarbonsäure, 3,5-Pyrazoldicarbonsäure, 2-Nitrobenzol-1,4-dicarbonsäure, Heptan-1,7-dicarbonsäure, Cyclobutan-1,1-dicarbonsäure 1,14-Tetradecandicarbonsäure, 5,6-Dehydronorbornan-2,3-dicarbonsäure oder 5-Ethyl-2,3-Pyridindicarbonsäure,
Tricarbonsäuren wie etwa

2-Hydroxy-1,2,3-propantricarbonsäure, 7-Chlor-2,3,8-chinolintricarbonsäure, 1,2,4-Benzoltricarbonsäure, 1,2,4-Butantricarbonsäure, 2-Phosphono-1,2,4-butantricarbonsäure, 1,3,5-Benzoltricarbonsäure, 1-Hydroxy-1,2,3-Propantricarbonsäure, 4,5-Dihydro-4,5-dioxo-1H-pyrrolo[2,3-F]chinolin-2,7,9-tricarbonsäure, 5-Acetyl-3-amino-6-methylbenzol-1,2,4-tricarbonsäure, 3-Amino-5-benzoyl-6-methylbenzol-1,2,4-tricarbonsäure, 1,2,3-Propantricarbonsäure oder Aurintricarbonsäure,
oder Tetracarbonsäuren wie etwa
1,1-Dioxidperylo[1,12-BCD]thiophen-3,4,9,10-tetracarbonsäure, Perylentetracarbonsäuren wie Perylen-3,4,9,10-tetracarbonsäure oder Perylen-1,12-sulfon-3,4,9,10-tetracarbonsäure, Butantetracarbonsäuren wie 1,2,3,4-Butantetracarbonsäure oder Meso-1,2,3,4-Butantetracarbonsäure, Decan-2,4,6,8-tetracarbonsäure, 1,4,7,10,13,16-Hexaoxacyclooctadecan-2,3,11,12-tetracarbonsäure, 1,2,4,5-Benzoltetracarbonsäure, 1,2,11,12-Dodecantetracarbonsäure, 1,2,5,6-Hexantetracarbonsäure, 1,2,7,8-Octantetracarbonsäure, 1,4,5,8-Naphthalintetracarbonsäure, 1,2,9,10-Decantetracarbonsäure, Benzophenontetracarbonsäure, 3,3',4,4'-Benzophenontetracarbonsäure, Tetrahydrofurantetracarbonsäure oder Cyclopentantetracarbonsäuren wie Cyclopentan-1,2,3,4-tetracarbonsäure
zu nennen.

Ganz besonders bevorzugt werden gegebenenfalls mindestens einfach substituierte mono-, di-, tri-, tetra- oder höherkernige aromatische Di-, Tri- oder Tetracarbonsäuren eingesetzt, wobei jeder der Kerne mindestens ein Heteroatom enthalten kann, wobei zwei oder mehr Kerne gleiche oder unterschiedliche Heteroatome enthalten kann. Beispielsweise bevorzugt werden monokernige Dicarbonsäuren, monokernige Tricarbonsäuren, monokernige Tetracarbonsäuren, dikernige Dicarbonsäuren, dikernige Tricarbonsäuren, dikernige Tetracarbonsäuren, trikernige Dicarbonsäuren, trikernige Tricarbonsäuren, trikernige Tetracarbonsäuren, tetrakernige Dicarbonsäuren, tetrakernige Tricarbonsäuren und/oder tetrakernige Tetracarbonsäuren. Geeignete Heteroatome sind beispielsweise N, O, S, B, P, Si, Al, bevorzugte Heteroatome sind hierbei N, S und/oder O. Als geeigneter Substituent ist diesbezüglich unter anderem -OH, eine Nitrogruppe, eine Aminogruppe oder eine Alkyl- oder Alkoxygruppe zu nennen.

Insbesondere bevorzugt werden als mindestens zweizähnige organische Verbindungen Acetylendicarbonsäure (ADC), Benzoldicarbonsäuren, Naphthalindicarbonsäuren, Biphenyldicarbonsäuren wie beispielsweise 4,4'-Biphenyldicarbonsäure (BPDC), Bipyridindicarbonsäuren wie beispielsweise 2,2'-Bipyridindicarbonsäuren wie beispielsweise 2,2'-Bipyridin-5,5-dicarbonsäure, Benzoltricarbonsäuren wie beispielsweise 1,2,3-Benzoltricarbonsäure oder 1,3,5-Benzoltricarbonsäure (BTC), Adamantantetracarbonsäure (ATC). Adamantandibenzoat (ADB) Benzoltribenzoat (BTB), Methantetrabenzoat (MTB), Adamantantetrabenzoat oder Dihydroxyterephthalsäuren wie beispielsweise 2,5-Dihydroxylerephthalsäure (DHBDC) eingesetzt.

Ganz besonders bevorzugt werden unter anderem lsophtalsäure, Terephthalsäure, 2,5-Dihydroxyterephthalsäure, 1,2,3-Benzoltricarbonsäure, 1,2,4-Benzoltricarbonsäure, 1,3,5-Benzoltricarbonsäure, Fumarsäure, Bernsteinsäure, Maleinsäure, Glutarsäure, 2,6-Naphthalindicarbonsäure, 1,2,4,5-Benzoltetracarbonsäure, Citronensäure, Weinsäure oder Oxalsäure eingesetzt. Insbesondere bevorzugt sind Oxalsäure, Isophthalsäure oder Terephthalsäure.

Es ist bevorzugt, wenn die mindestens eine mindestens zweizähnige organische Verbindung nur aus den Elementen Kohlenstoff, Wasserstoff und Sauerstoff aufgebaut ist. Hierbei ist bevorzugt, dass das molare Verhältnis C : 0 ≤ 3, mehr bevorzugt ≤ 2 ist.

Neben diesen mindestens zweizähnigen organischen Verbindungen kann der MOF auch eine oder mehrere einzähnige Liganden umfassen.

Geeignete Lösemittel zur Herstellung der MOF sind unter anderem Ethanol, Dimethylformamid, Toluol, Methanol, Chlorbenzol, Diethylformamid, Dimethylsulfoxid, Wasser, Wasserstoffperoxid, Methylamin, Natronlauge, N-Methylpolidonether, Acetonitril, Benzylchlorid, Triethylamin, Ethylenglykol und Gemische hiervon. Weitere Metallionen, mindestens zweizähnige organische Verbindungen und Lösemittel für die Herstellung von MOF sind unter anderem in US-A 5,648,508 oder DE-A 101 11 230 beschrieben.

Die metallorganischen Gerüstmaterialien für das erfindungsgemäße Verfahren enthalten Poren, insbesondere Mirko- und/oder Mesoporen. Mikroporen sind definiert als solche mit einem Durchmesser von 2 nm oder kleiner und Mesoporen sind definiert durch einen Durchmesser im Bereich von 2 bis 50 nm, jeweils entsprechend nach der Definition, wie sie Pure Applied Chem. 57 (1985), Seiten 603-619, insbesondere auf Seite 606 angegeben ist. Die Anwesenheit von Mikro- und/oder Mesoporen kann mit Hilfe von Sorptionsmessungen überprüft werden, wobei diese Messungen die Aufnahmekapazität der metallorganischen Gerüstmaterialien für Stickstoff bei 77 Kelvin gemäß DIN 66131 und/oder DIN 66134 bestimmt.

Vorzugsweise beträgt die spezifische Oberfläche - berechnet nach dem Langmuir-Modell (DIN 66131, 66134) für ein MOF in Pulverform bei mehr als 5 m²/g, mehr bevorzugt über 10 m²/g, mehr bevorzugt mehr als 50 m²/g, weiter mehr bevorzugt mehr als 500 m²/g, weiter mehr bevorzugt mehr als 1000 m²/g und besonders bevorzugt mehr als 1500 m²/g.

Formkörper aus metallorganischen Gerüstmaterialien können eine niedrigere spezifische Oberfläche besitzen; vorzugsweise jedoch mehr als 10 m²/g, mehr bevorzugt mehr als 50 m²/g, weiter mehr bevorzugt mehr als 500 m²/g, insbesondere mehr als 1000 m²/g.

Die Porengröße des porösen metallorganischen Gerüstmaterials kann durch Wahl des geeigneten Liganden und/oder der mindestens zweizähnigen organischen Verbindung gesteuert werden. Allgemein gilt, dass je größer die organische Verbindung desto grö-βer die Porengröße ist. Vorzugsweise beträgt die Porengröße von 0,2 nm bis 30 nm, besonders bevorzugt liegt die Porengröße im Bereich von 0,3 nm bis 3 nm bezogen auf das kristalline Material.

In einem Formkörper des metallorganischen Gerüstmaterials treten jedoch auch größere Poren auf, deren Größenverteilung variieren kann. Vorzugsweise wird jedoch mehr als 50 % des gesamten Porenvolumens, insbesondere mehr als 75 %, von Poren mit einem Porendurchmesser von bis zu 1000 nm gebildet. Vorzugsweise wird jedoch ein Großteil des Porenvolumens von Poren aus zwei Durchmesserbereichen gebildet. Es ist daher weiter bevorzugt, wenn mehr als 25 % des gesamten Porenvolumens, insbesondere mehr als 50 % des gesamten Porenvolumens von Poren gebildet wird, die in einem Durchmesserbereich von 100 nm bis 800 nm liegen und wenn mehr als 15 % des gesamten Porenvolumens, insbesondere mehr als 25 % des gesamten Porenvolumens von Poren gebildet wird, die in einem Durchmesserbereich von bis zu 10 nm liegen. Die Porenverteilung kann mittels Quecksilber-Porosimetrie bestimmt werden.

Die Herstellung von Formkörpern aus metallorganischen Gerüstmaterialien ist beispielsweise in WO-A 03/102000 beschrieben.

Da die Gerüststruktur des metallorganischen Gerüstmaterials zumindest teilweise beibehalten wird, ist es bevorzugt, wenn - und insofern dies der Fall ist - die spezifische Oberfläche (N₂ nach Langmuir) von metallorganischem Gerüstmaterial zu Oxid vorzugsweise < 50 : 1, besonders bevorzugt < 20 : 1, weiter besonders bevorzugt < 15 : 1 und im Falle von metallorganischen Gerüstmaterialien aus Hauptgruppenmetallen insbesondere < 5 : 1 und weiterhin mehr bevorzugt < 4 : 1 ist.

Für den Fall, dass das Metalloxid Al₂O₃ ist, wird bevorzugt, wenn die spezifische Oberfläche mindestens 400 m²/g (N₂ nach Langmuir) beträgt.

Für den Fall, dass das Metalloxid MgO ist, wird bevorzugt, wenn dessen spezifische Oberfläche mindestens 100 m²/g (N₂ nach Langmuir) beträgt.

Für den Fall, dass das Metalloxid ZrO₂ ist, wird bevorzugt, wenn dessen spezifische Oberfläche mindestens 50 m²/g (N₂ nach Langmuir) ist.

Die Metalloxide können jeweils in der Form anfallen, wie das metallorganische Gerüstmaterial eingesetzt wurde. Vorzugsweise wird das metallorganische Gerüstmaterial als Pulver eingesetzt.

Die Metalloxide, welche nach dem erfindungsgemäßen Verfahren erhältlich sind, können prinzipiell für sämtliche Anwendungen eingesetzt werden, welche für konventionell erhaltene Metalloxide bekannt sind.

Insbesondere sind solche Anwendungen von Interesse, bei denen eine möglichst hohe spezifische Oberfläche von Vorteil ist.

Beispielhafte Verwendungen eines erfindungsgemäßen Metalloxids sind solche, wobei das Metalloxid als keramischer Werkstoff, als Katalysator, insbesondere Photokatalysator, Pigment, beispielsweise Lichtschutzpigment, Träger, beispielsweise als Katalysatorträger, Adsorbens, beispielsweise zur Speicherung oder Trennung von Stoffen, insbesondere von Flüssigkeiten oder Gasen, als Isolationsmaterial, Schleifmittel, Hilfs - oder Füllstoff eingesetzt wird.

### Beispiele

### Beispiel 1: Herstellung von Aluminiumoxid

23,9 kg Terephthalsäure (BDC) und 28 kg Al₂(SO₄)₃*18H₂O werden in 150 kg DMF suspendiert und die Mischung 24 Stunden bei 130°C gerührt. Anschließend wird der Feststoff abfiltriert, mit 4 x 10 kg Methanol gewaschen und mit N₂ während 96 Stunden trockengestrippt.

Hierbei ergibt sich eine Oberfläche (bestimmt mit N₂ nach Langmuir) von 1381 m²/g.

Das so erhaltene Gerüstmaterial in Form eines Pulvers wird ca. 24 h in einem Muffelofen bei etwa 475 bis 500°C in einer Luftatmosphäre calciniert. Dabei ist der Kohlenstoff fast quantitativ entfernt (Restgehalt 0,35 Gew.%). Das Produkt ist ein amorphes Aluminiumoxid mit einer N₂-Oberfläche von 452 m²/g (Langmuir).

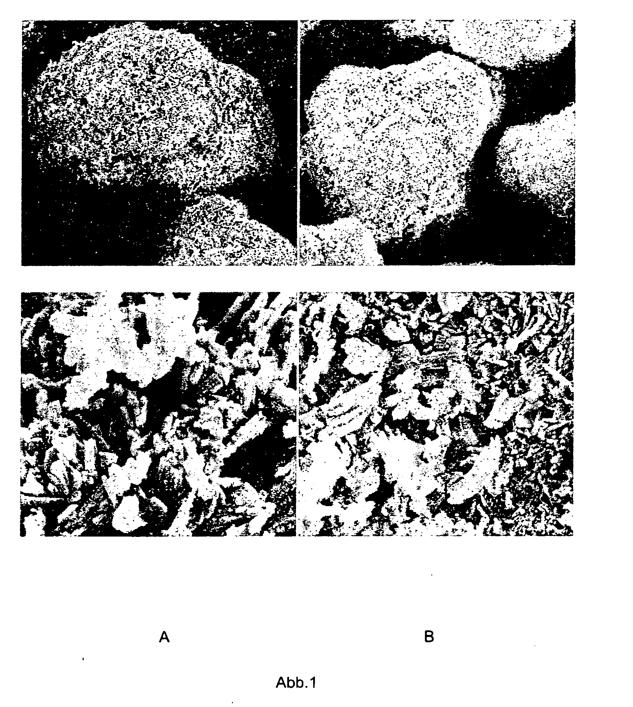

Abbildung 1 zeigt die Elektronenmikroskopie (SEM) des metallorganischen Gerüstmaterials (A) und des Oxids (B), wobei in der oberen Bildhälfte ein Maßstab von 1000:1 und in der unteren Hälfte ein Maßstab von 20000:1 vorliegt. Wie Abbildung 1 zeigt, bleibt die Morphologie der ursprünglichen Partikel weitgehend erhalten.

### Vergleichsbeispiel 2

Im Vergleich zu Beispiel 1 wird die spezifische Oberfläche von konventionell hergestelltem und kommerziell erhältlichem Aluminiumoxid bestimmt. Hierbei ergab sich für die Proben Versal 200 (UOP LLC, Des Plaines, US), Spheralite (Procatalyse, Usine de Salindre, FR), Al₂O₃ der Fa. Alcoa Inc. (Pittsburgh, US), Versal 250 (Eurosupport, KH Amersfoort, NL) ein Wert für die spezifische Oberfläche im Bereich von etwa 320 bis 350 m²/g.

### Beispiel 3: Herstellung von Magnesiumoxid

10,984 g Magnesiumnitrat*6(H₂O) werden in 68,5 g DEF gelöst. In einem Autoklavenbecher (Teflon-Liner) werden 6,657 g 2,6-Naphthalindicarbonsäure in 68,5 g DEF suspendiert. Anschließend wird die magnesiumsalzhaltige Lösung zugegeben und für 10 Min. verrührt. Die Synthese erfolgt anschließend im geschlossenen Autoklav bei 105°C während 24 Stunden. Die gelben Kristalle werden abfiltriert. Der Filterkuchen wird mit 250 ml DMF aufgeschlämmt und für 20 Min. gerührt. Das Produkt wird abfiltriert, danach zuerst mit DMF und anschließend mit Chloroform gewaschen. Anschließend wird an der Luft getrocknet.

Die N₂-Oberfläche (nach Langmuir) eines derart hergestellten Mg-Naphthalindicarbonsäure-MOFs liegt typischerweise im Bereich von 80 bis 120 m²/g.

Der Magnesium-2,6-Naphthalindicarbonsäure-MOF wird 5 Stunden bei 650°C calciniert. Das Produkt ist ein Magnesiumoxid mit einer N₂-Oberfläche von 133 m²/g (Langmuir).

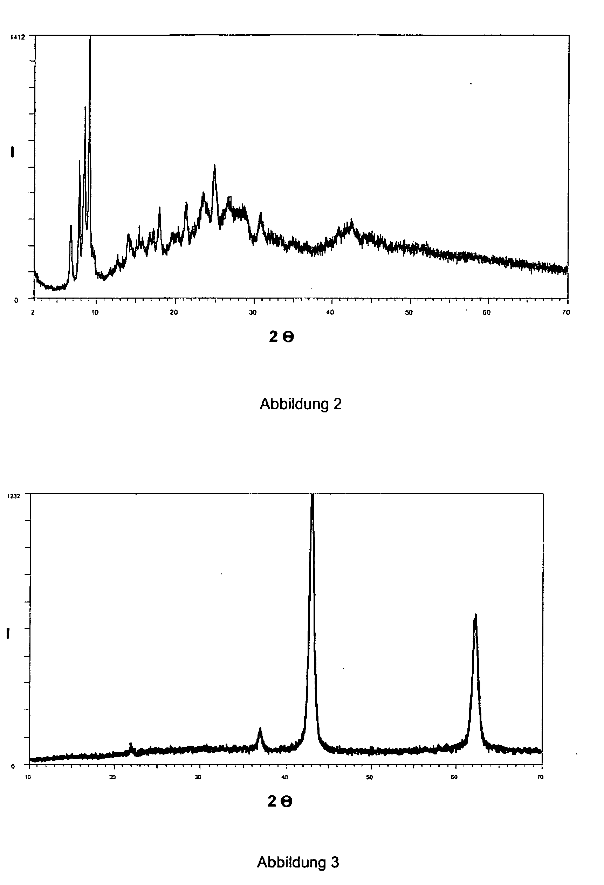

Die Abbildungen 2 und 3 zeigen das Röntgendiffraktogramm (XRD) von Gerüstmaterial (Abb. 2) und Oxid (Abb. 3), wobei 1 die Intensität (Lin (courts)) angibt und 2 Θ die 2-Theta-Skala beschreibt.

Die Morphologie der ursprünglichen Partikel bleibt ebenfalls weitgehend erhalten.

### Beispiel 4: Herstellung von Zirkonoxid

5 g ZrOCl₂ und 9,33 g Terephthalsäure werden in 300 ml DMF in einem Glaskolben 17 h bei 130°C unter Rückfluss gerührt. Der Niederschlag wird abfiltriert, mit 3 x 50 ml DMF und 4 x 50 ml Methanol gewaschen und 4 Tage im Vakuumtrockenschrank bei 150°C vorgetrocknet. Abschließend wird 2 Tage lang in einem Muffelofen bei 275°C calciniert (100 l/h Luft). Es werden 5,17 g eines braunen Materials erhalten.

Es ergibt sich eine N₂-Oberfläche von 836 m²/g (Langmuir).

Es wird 48 h bei 500°C calciniert.

Das Produkt ist ein Zirkonoxid mit einer N₂-Oberfläche von 61 m²/g (Langmuir). Die Verteilung der Porendurchmesser bleibt im Wesentlichen erhalten.

### Beispiel 5: Herstellung eines gemischten Al/Zr-Oxids

Aus 5 g AlCl₃*6H₂O, 2,25 g ZrOCl₂ und 8,14 g Terephthalsäure wird durch Fällung in 300 ml DMF (130°C, 17 h) ein Zr-dotierter Al-Terephthalsäure-MOF hergestellt. Nach Filtrieren und Waschen mit DMF und Methanol wird dieser zunächst bei 150°C im Vakuumtrockenschrank vorgetrocknet und anschließend bei 330°C 48 h in einem Muffelofen unter Luftzufuhr calciniert. Der MOF weist eine N₂-Oberfläche von 1448 m²/g auf (Langmuir) und enthält neben 8,5 Gew.-% Al auch 9,8 Gew.-% Zr.

Der MOF-Precursor wird 48 h bei 500°C calciniert und so in ein gemischtes Al/Zr-Oxid umgewandelt. Das Produkt hat eine Langmuir-Oberfläche von 358 m²/g.

### Vergleichsbeispiel 6: Herstellung von Zinkoxid

96,7 g Zn(NO₃)₂*4H₂O und 20,8 g Terephthalsäure werden in 2825 g DEF suspendiert. Das Reaktionsgemisch wird 3,5 Stunden bei 130°C gehalten. Nach dem Abkühlen wird der Feststoff abfiltriert und mit 4 x 500 ml wasserfreiem Aceton gewaschen. Der Feststoff wird zunächst bei Raumtemperatur im Stickstoffstrom 2 bis 4 Tage vorgetrocknet und anschließend im Vakuumtrockenschrank 16 Stunden evakuiert (≤ 1 mbar).

Der Zink-Terephthalsäure-MOF (MOF-5) weist eine N₂-Oberfläche von 2811 m²/g (Langmuir) auf.

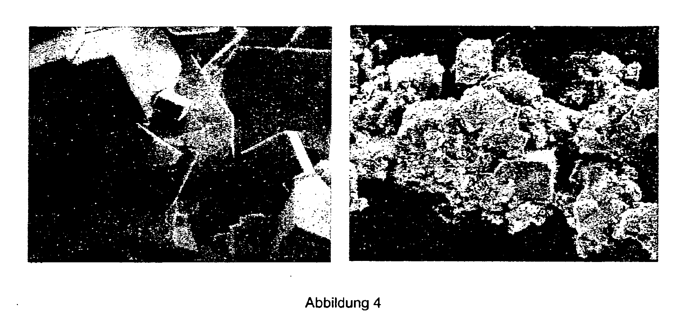

Das erhaltene Pulver wird 16 h bei 500°C calciniert. Das Produkt ist ein Zinkoxid mit einer N₂-Oberfläche von nur noch 20 m²/g (Langmuir). Abbildung 4 zeigt die Elektronenmikroskopaufnahme (SEM) von MOF-5 (links) und Oxid (rechts) bei einer Auflösung von 500 : 1. Es ist zu erkennen, dass die MOF-Struktur sich größtenteils zersetzt hat.

### Beispiel 7 Herstellung eines Aluminiumoxids

27,8 g Al(NO₃)₃*9H₂O und 4,3 g Fumarsäure werden in 520,5 g DMF suspendiert und 3 Tage bei 130°C im Glaskolben gerührt. Das Produkt wird abfiltriert und mit 2 x 100 ml DMF und 4 x 100 ml MeOH nachgewaschen. Es werden 4,5 g eines AI-Fumarsäure-MOFs mit einer N₂-Oberfläche von 776 m²/g (Langmuir) erhalten. Nach Tempern in Luft bei 500°C wird ein Aluminiumoxid mit einer Oberfläche von 510 m²/g erhalten.

## Patentansprüche

1. Verfahren zur Herstellung eines Metalloxids den Schritt enthaltend
- Erhitzen eines porösen metallorganischen Gerüstmaterials, wobei das Gerüstmaterial mindestens eine an mindestens ein Metallion koordinativ gebundene, mindestens zweizähnige organische Verbindung enthält und das Metallion ausgewählt ist aus den Metallen bestehend aus den Gruppen 2. bis 4. und 13. des Periodensystems der Elemente, über die vollständige Zersetzungstemperatur des Gerüstmaterials.

2. Verfahren nach Anspruch 1, **dadurch gekennzeichnet, dass** das Metallion ausgewählt ist aus der Gruppe der Metalle bestehend aus Aluminium. Magnesium, Titan und Zirkonium.

3. Verfahren nach Anspruch 1 oder 2, **dadurch gekennzeichnet, dass** das Gerüstmaterial nur aus einem Metall oder in dotierter Form aufgebaut ist.

4. Verfahren nach einem der Ansprüche 1 bis 3, **dadurch gekennzeichnet, dass** das Erhitzen in Gegenwart einer oxidierenden Atmosphäre mit einem Sauerstoff liefernden Bestandteil erfolgt.

5. Verfahren nach Anspruch 4, **dadurch gekennzeichnet, dass** der Sauerstoff liefernde Bestandteil während des Erhitzens erhöht wird.

6. Verfahren nach einem der Ansprüche 1 bis 5, **dadurch gekennzeichnet, dass** die vollständige Zersetzungstemperatur im Bereich von 250 °C bis 1000 °C liegt.

7. Verfahren nach einem der Ansprüche 1 bis 6, **dadurch gekennzeichnet, dass** die mindestens eine mindestens zweizähnige organische Verbindung sich von einer Di-, Tri- oder Tetracarbonsäure ableitet.

## Claims

1. A method for producing a metal oxide comprising the step
- heating a porous metal-organic framework material, the framework material comprising at least one at least bidentate organic compound bound to at least one metal ion by coordination, and the metal ion being selected from the metals comprising groups 2 to 4 and 13 of the Periodic Table of the Elements, above the complete decomposition temperature of the framework material.

2. The method according to claim 1, wherein the metal ion is selected from the group of metals consisting of aluminum, magnesium, titanium and zirconium.

3. The method according to claim 1 or 2, wherein the framework material is made up only of one metal, or is in doped form.

4. The method according to one of claims 1 to 3, wherein the heating proceeds in the presence of an oxidizing atmosphere having an oxygen-supplying component.

5. The method according to claim 4, wherein the oxygen-supplying component is increased during heating.

6. The method according to one of claims 1 to 5, wherein the complete decomposition temperature is in the range from 250°C to 1000°C.

7. The method according to one of claims 1 to 6, wherein the at least one at least bidentate organic compound is derived from a dicarboxylic, tricarboxylic or tetracarboxylic acid.

## Revendications

1. Procédé pour la préparation d'un oxyde métallique, comportant l'étape
- chauffage d'une matière matrice organométallique poreuse, la matière matrice contenant au moins un composé organique au moins bidenté lié par coordination à au moins un ion métallique et l'ion métallique étant choisi parmi les métaux des groupes 2 à 4 et 13 du système périodique des éléments, au-dessus de la température de décomposition totale de la matière matrice.

2. Procédé selon la revendication 1, **caractérisé en ce que** l'ion métallique est choisi dans le groupe des métaux consistant en aluminium, magnésium, titane et zirconium.

3. Procédé selon la revendication 1 ou 2, **caractérisé en ce que** la matière matrice est constituée seulement d'un métal ou sous forme dopée.

4. Procédé selon l'une quelconque des 1 à 3, **caractérisé en ce que** le chauffage s'effectue en présence d'une atmosphère oxydante avec un composant fournissant de l'oxygène.

5. Procédé selon la revendication 4, **caractérisé en ce que** le composant fournissant de l'oxygène est augmenté au cours du chauffage.

6. Procédé selon l'une quelconque des 1 à 5, **caractérisé en ce que** la température de décomposition totale se situe dans la plage de 250 °C à 1 000 °C.

7. Procédé selon l'une quelconque des 1 à 6, **caractérisé en ce que** ledit au moins un composé organique au moins bidenté dérive d'un acide di-, tri- ou tétracarboxylique.
